# EUROPEAN PATENT SPECIFICATION

(11) **EP 2 004 209 B1**
(45) Date of publication and mention of the grant of the patent: **24.07.2013**
(21) Application number: 07754554.9
(22) Date of filing: 03.04.2007
(51) Int. Cl.: A61K 38/16, A61K 38/17, C12N 9/10, A61K 38/39, C07K 14/47, A61K 38/45

(54) **COMPOSITION AND METHOD FOR STABILIZING CORNEAL TISSUE AFTER REFRACTIVE SURGERY**
ZUSAMMENSETZUNG UND VERFAHREN ZUR STABILISIERUNG DES HORNHAUTGEWEBES NACH EINEM REFRAKTIVEN CHIRURGISCHEN EINGRIFF
COMPOSITION ET PROCÉDÉ DE STABILISATION DE TISSU DE LA CORNÉE APRÈS UNE CHIRURGIE RÉFRACTIVE

(30) Priority: 13.04.2006 US 791413 P
(43) Date of publication of application: 24.12.2008
(73) Proprietor: Euclid Systems Corporation, Herndon, VA 20171 (US)
(72) Inventor: DEWOOLFSON, Bruce, Vienna, VA 22182 (US); DEVORE, Dale, Chelmsford, MA 01824 (US); THOMPSON, Vance, Sioux Falls, SD 57105 (US)
(74) Representative: Gill, David Alan
(86) International application number: PCT/US2007/008049
(87) International publication number: WO 2007/120457

(56) References cited:
- US-A1- 2004 001 821
- US-B1- 6 444 791
- US-B1- 6 520 956
- US-B1- 6 946 440
- US-B1- 6 946 440
- WOLLENSAK G ET AL: "Riboflavin/ultraviolet-A-induced collagen crosslinking for the treatment of keratoconus" AMERICAN JOURNAL OF OPHTHALMOLOGY, OPHTHALMIC PUBL, CHICAGO, IL, US, vol. 135, no. 5, 1 May 2003 (2003-05-01), pages 620-627, XP002496965 ISSN: 0002-9394
- WOLLENSAK G ET AL: "[Treatment of keratoconus by collagen cross linking]" January 2003 (2003-01), DER OPHTHALMOLOGE : ZEITSCHRIFT DER DEUTSCHEN OPHTHALMOLOGISCHEN GESELLSCHAFT JAN 2003, VOL. 100, NR. 1, PAGE(S) 44 - 49 , XP002569416 ISSN: 0941-293X * abstract * * page 46 *
- WOLLENSAK ET AL.: 'Riboflavin/Ultraviolet-A-Induced Collagen Crosslinking for the Treatment of Keratoconus' AMERICAN JOURNAL OF OPTHAMOLOGY vol. 135, 2003, pages 620 - 627, XP002496965
- SPOERL ET AL.: 'Induction of Cross-links in Corneal Tissue' EXPERIMENTAL EYE RESEARCH vol. 66, 1998, pages 97 - 103, XP008107083

## Description

This application claims priority to U.S. Provisional Patent Application No. 60/791,413 filed on April 13, 2006.

### DESCRIPTION OF THE INVENTION

### Field of the Invention

The present invention relates to methods of stabilizing collagen fibrils in the cornea. These methods can be used to improve the outcome following refractive surgery, and to treat conditions of the cornea such as keratectasia and keratoconus.

### Background of the Invention

The cornea is the first and most powerful refracting surface of the optical system of the eye. It is made up of five layers, the outermost of which is the epithelium. The epithelium is only four to five cells thick, and renews itself continuously. Underneath the epithelium is the acellular Bowman's membrane. It is composed of collagen fibrils and normally transparent. Below Bowman's membrane is the stroma. The stroma makes up approximately 90% of the cornea's thickness. This middle layer is mostly water (78%) and collagen (16%), although other proteoglycans and glycoproteins are also present. Descemet's membrane, which lies below the stroma, is also composed of collagen fibers, but of a different type than that found in the stroma. The endothelium ties beneath Descemet's membrane. It is a single layer of flattened, non-regenerating cells and functions to pump excess fluid out of the stroma.

When the cornea is misshapen or injured, vision impairment can result. In the case of a misshapen cornea, eyeglasses and contact lenses have traditionally been used to correct refractive errors, but refractive surgical techniques are now also routinely used. There are currently several different techniques in use. In radial keratotomy (RK), several deep incisions are made in a radial pattern around the cornea, so that the central portion of the cornea flattens. Although this can correct the patient's vision, it also weakens the cornea, which may continue to change shape following the surgery. Photorefractive keratectomy (PRK) is another technique. It uses an excimer laser to sculpt the surface of the cornea. In this procedure, the epithelial basement membrane is removed, and Bowman's membrane and the anterior stroma photoablated. However, regression and corneal haze can occur following PRK, and the greater the correction attempted, the greater the incidence and severity of the haze.

Laser in situ keratomileusis (LASIK) is yet another alternative. In this technique, an epithelial-stromal flap is cut with a microkeratome. The flap is flipped back on its hinge, and the underlying stroma ablated. The flap is then reseated. There is a risk that the flap created will later dislodge, however. In addition, the CRS-USA LASIK Study noted that overall, 5.8% of LASIK patients experienced complications at the three-month follow up period that did not occur during the procedure itself. These complications included corneal edema (0.6%), corneal scarring (0.1%), persistent epithelial defect (0.5%), significant glare (0.2%), persistent discomfort or pain (0.5%), interface epithelium (0.6%), cap thinning (0.1 %) and interface debris (3.2%).

Most patients will have stable results after LASIK. That is, the one month to three month results will usually be permanent for most patients. However, some patients with initially good results may experience a change in their refraction over the first 3 to 6 months (and possibly longer). This shift in results over time is called regression. LASIK results in regression and haze less frequently than does PRK, presumably because it preserves the central corneal epithelium.

The chance of having regression following LASIK is related to the initial amount of refractive error: patients with higher degrees of myopia (-8.00 to - 14.00) are more likely to experience regressions. For example, a -10.00 myope may initially be 20/20 after LASIK at the 2 week follow-up visit. However, over the course of the next 3 months, the refractive error may shift (regress) from -0.25 to-1.50 (or even more). This could reduce one's visual acuity without glasses to less than 20/40, a point at which the patient would consider having an enhancement.

All surgical procedures involve varying degrees of traumatic injury to the eye and a subsequent wound healing process. Netto et al., Cornea, Vol. 24, pp. 509-22 (2005). In addition, they reduce the eye's biomechanical rigidity, and postoperative keratectasia can result. Keratectasia is an abnormal bulging of the cornea. In keratectasia, the posterior stroma thins, possibly due to interruption of the crosslinks of collagen fibers and/or altered proteoglycans composition, reducing the stiffness of the cornea and permitting it to shift forward. Dupps, W.J., J. Refract. Surg., Vol. 21, pp. 186-90 (2005). The forward shift in the cornea causes a regression in the refractive correction obtained by the surgical procedure. The incidence of keratectasia following LASIK is estimated to be 0.66% (660 per 100,000 eyes) in eyes having greater than -8 diopters of myopia preoperatively. Pallikaris et al., J. Cataract Refract. Surg., Vol. 27, pp. 1796-1802 (2001). Although at present keratectasia is a rare complication of refractive surgery, the number of procedures each year continues to increase, so that even a rare condition will impact many individuals. T. Seiler, J. Cataract Refract. Surg., Vol. 25, pp. 1307-08 (1999).

Keratoconus is another condition in which the rigidity of the cornea is decreased. Its frequency is estimated at 4-230 per 100,000. Clinically, one of the earliest signs of keratoconus is an increase in the corneal curvature, which presents as irregular astigmatism. The increase in curvature is thought to be due to stretching of the stromal layers. In advanced stages of keratoconus, a visible cone-shaped protrusion forms which is measurably thinner than surrounding areas of the cornea.

Keratoconus may involve a general weakening of the strength of the cornea, which eventually results in lesions in those areas of the cornea that are inherently less able to withstand the shear forces present within the cornea. Smolek et al., Invest. Ophthalmol. Vis. Sci. Vol. 38, pp. 1289-90 (1997). Andreassen et al., Exp. Eye Res., Vol. 31, pp. 435-41 (1980), compared the biomechanical properties of keratoconus and normal corneas and found a 50% decrease in the stress necessary for a defined strain in the keratoconus corneas. The alterations in the strength of the cornea in keratoconus appear to involve both the collagen fibrils and their surrounding proteoglycans. For example, Daxer et al., Invest. Ophthalmol. & Vis. Sci., Vol. 38, pp. 121-29 (1997), observed that in normal cornea, the collagen fibrils were oriented along horizontal and vertical directions that correspond to the insertion points of the four musculi recti oculi. In keratoconus corneas, however, that orientation of collagen fibrils was lost within the diseased areas. In addition, Fullwood et al., Biochem. Soc. Transactions, Vol. 18, pp. 961-62 (1990), found that there is an abnormal arrangement of proteoglycans in the keratoconus cornea, leading them to suggest that the stresses within the stroma may cause slipping between adjacent collagen fibrils.

Because both keratoconus and postoperative keratectasia involve reduced corneal rigidity, relief from each disease could be provided by methods of increasing the rigidity of the cornea. For example, methods which increase the rigidity of the cornea can be used to treat postoperative keratectasia. Optionally, the treatment can be administered to a patient who plans to undergo a refractive surgical procedure as a prophylactic therapy. In other cases, the treatment can be administered during the surgical procedure itself. In still other situations, the treatment may not be initiated until after the refractive surgical procedure. Of course, various combinations of treatment before, during, and after the surgery are also possible.

It has also been suggested that a therapeutic increase in the stiffness of the cornea could delay or compensate for the softening of the cornea that occurs in keratoconus. Spoerl et al., Exp. Eye Res., Vo. 66, pp. 97-103 (1998). While acknowledging that the basis for the differences in elasticity between normal and keratoconus corneas is unknown, those authors suggest that a reduction in collagen crosslinks and a reduction in the molecular bonds between neighboring stromal proteoglycans could play a role.

As discussed below, the methods of increasing corneal rigidity and compensating for corneal softness that currently exist suffer from drawbacks that include development of corneal haze and scarring, and the risk of endothelial cell damage. These drawbacks are associated with the particular agents used in the methods. The need exists, therefore, for alternate methods of providing collagen crosslinks to increase the rigidity of the cornea.

The organization of collagen fibrils is the key to the cornea's transparency, and the arrangement of the collagen lamellae is the basis of its shape and strength. Meeks & Boote, Exp. Eye Res., Vol. 78, pp. 503-12 (2004), provide a recent review of the organization of the collagen fibrils and their associated proteoglycans. Each collagen fibril is made up of some 250 collagen molecules. Unlike collagen in other tissues, however, the axial periodicity is 65 nm rather than the usual 67 nm. The fibril diameter is approximately 31 nm, and each fibril is spaced on average about 62 nm apart, although this spacing varies, increasing from the central cornea towards the limbus. The fibrils are themselves organized into a lattice, but while early investigators predicted that the collagen fibrils would pack in a perfect lattice in the stroma, more recent studies have found that there are multiple lattices, each at most three fibril diameters.

Type I collagen is the predominant collagen within the fibrils, although types III, IV, V, VI, and XII are also present. Type VI collagen forms filaments that that run between the corneal fibrils and may interact with proteoglycans in the interfibrillar matrix to stabilize the fibrils. Proteoglycans also associate with the other collagen fibrils. There are two types of proteoglycans: chondroitin/dermatan-sulphate-containing and keratan sulphate containing. Decorin is the only molecule of the first type, whereas there are three keratan sulphate containing proteoglycans: lumican, keratocan, and mimecan. Recent studies suggest that the three-dimensional arrangement involves a backbone of collagen fibrils enwrapped by a ring-like structure of proteoglycans which interconnect next-nearest neighbor collagen fibrils to form a lamella. Muller et al., Exp. Eye Res., Vol. 78, pp. 493-501 (2004).

The collagen fibrils in the scar tissue that forms following refractive surgery is disordered, resulting in corneal cloudiness. Kaji et al., J. Cataract Refract. Surg., Vol. 24, pp., 1441-46 (1998). In most patients this scar tissue heals over time. As the scar heals, the collagen fibrils become regular in size and orientation, and the proteoglycans content returns to normal. In keratoconus, the collagen fibrils are also disordered. A recent study suggests that the lamellae unravel from their limbal anchors, much like a piece of cloth rips starting from a tear in the edge. Meek et al., Invest. Ophthalmol. & Vis. Sci, Vol. 46, pp. 1948-56 (2005). Those authors also propose that part of this breakdown is triggered by a defect in the interfibrillar matrix that stabilizes the collagen fibrils, resulting in lamellar or fibrillar slippage.

Wollensak et al., J. Cataract Refract. Surg., Vol. 29, pp. 1780-85 (2003), have shown that the rigidity of the cornea can be improved by cross-linking the collagen fibers present in the cornea with the non-protein agent riboflavin. In their method, they apply a photosensitizing solution containing riboflavin to the cornea, then ultraviolet A (UVA) irradiation. This treatment forms collagen crosslinks that increase the rigidity of the cornea. In one preliminary study, the progression of keratectasia in patients with keratoconus was reduced. Wollensak et al., Am. J. Ophthalmol., Vol. 135, pp. 620-27 (2003), Although no adverse effects were observed in that clinical study, the investigators have found evidence of endothelial cell damage in a rabbit model following riboflavin/UVA treatment. Wollensak et al., J. Cataract Refract. Surg., Vol. 23, pp. 1786-90 (2003). In addition, the treatment also has the undesirable effect of inducing keratocyte apoptosis. Wollensak et al., Cornea, Vol. 23, pp. 43-49 (2004).

Aldehydes have also been used to crosslink collagen fibers in the cornea. For example, U.S. Patent No. 6,537,545 describes the application of various aldehydes to a cornea in combination with a reshaping contact lens. The contact lens is used to induce the desired shape following either enzyme orthokeratology or refractive surgery, and the aldehyde is used to crosslink collagens and proteoglycans in the cornea. Spoerl & Seiler, J. Refract. Surg., Vol. 15, pp. 711-13 (1999), also tested the ability of several aldehydes to form collagen crosslinks. In application, however, aldehydes such as glutaraldehyde can lead to the development of corneal haze and scarring, while glyceraldehyde requires prolonged application times and its application is problematic. Wollensak et al., J. Cataract Refract. Surg., Vol. 29, pp. 1780-85 (2003).

Alternate methods of providing collagen crosslinks to increase the rigidity of the cornea are therefore needed.

It is accordingly an object of the invention to provide a method of stabilizing collagen fibrils. In one embodiment, the collagen fibrils are stabilized in association with a refractive surgical procedure. It is also an object of the invention to provide a method of treating keratectasia. The treatment can be prophylactic, contemporaneous with a surgical procedure, postoperative, or can involve multiple administrations during one or more of those time points. Another object of the invention is a method of treating keratoconus.
These objects are achieved by providing to a cornea decorin, a protein that crosslinks collagen fibrils. The protein can be provided in a pharmaceutically acceptable carrier.

### SUMMARY OF THE INVENTION

In accordance with the invention, there is provided decorin for use in improving an outcome following a refractive surgical procedure wherein:
a) a patient is to undergo a refractive surgical procedure and the decorin is administered to the eye of the patient before the procedure is carried out, and/or
b) the decorin is administered to the eye of the patient during a refractive surgical procedure, and/or
c) the decorin is administered to the eye of the patient following a refractive surgical procedure.

The decorin crosslinks collagen fibrils by binding to each of two different fibrils to form a bridge there between.

Optionally, the decorin may be for use in treating or preventing post-operative keratectasia.

The refractive surgical procedure may be one of radial keratotomy (RK), photorefractive keratectomy (PRK) or laser in-situ keratomileusis (LASIK).

Further optionally, the decorin may be used for preventing or treating keratectasia or keratoconus by administration to the eye of a patient who has keratectasia or keratoconus. The keratectasia may develop following a refractive surgical procedure and, in particular, may develop following laser in-situ keratomileusis (LASIK).

Optionally, the decorin may be glycosylated or unglycosylated.

Further optionally, the decorin may be recombinant human decorin.

Additional objects and advantages of the invention will be set forth in part in the description which follows, and in part will be obvious from the description, or may be learned by practice of the invention. The objects and advantages of the invention will be realized and attained by means of the elements and combinations particularly pointed out in the appended claims.

It is to be understood that both the foregoing general description and the following detailed description are exemplary and explanatory only and are not restrictive of the invention, as claimed.

### DESCRIPTION OF THE EMBODIMENTS

The inventors have found that collagen fibrils in the cornea can be stabilized by administering to the eye one or more proteins that crosslinks the collagen fibrils. In order that the present invention may be more readily understood, certain terms are first defined. Other definitions are set forth throughout the description of the embodiments.

### I. Definitions

A "refractive surgical procedure" includes, but is not limited to, Radial Keratotomy (RK), Photorefractive Keratoplasty (PRK), LASIK (Laser- Assisted In Situ Keratomileusis), Epi-LASIK, IntraLASIK, Laser Thermal Keratoplasty (LTK), and Conductive Keratoplasty.

"Stabilizing" includes increasing the rigidity, as measured by the Corneal Response Analyzer manufactured by Reichert Ophthalmic Institute. This instrument gives a quantitative measure of corneal rigidity called the Corneal Resistance Factor (CFR) and also a quantitative measure of corneal Historesis. "Stabilizing" can also mean decreasing the ability of one collagen fibril to move relative to another collagen fibril by virtue of increased intermolecular interactions.

"Crosslinks" includes the formation of both direct and indirect bonds between two or more collagen fibrils. Direct bonds include covalent bond formation between an amino acid in one collagen fibril and an amino acid in another fibril. For example, the transglutaminase family of enzymes catalyze the formation of a covalent bond between a free amine group (e.g., on a lysine) and the gamma-carboxamide group of glutamine. Transglutaminase thus is not itself part of the bond. Indirect bonds include those in which one or more proteins serve as an intermediary link between or among the collagen fibrils. For example, decorin is a horse-shoe shaped proteoglycan that binds to collagen fibrils in human cornea forming a bidentate ligand attached to two neighboring collagen molecules in the fibril or in adjacent fibrils, helping to stabilize fibrils and orient fibrillogenesis. Scott, JE, Biochemistry, Vol. 35, pages 8795 (1996).

A "protein that crosslinks collagen fibrils" includes proteins that form direct or indirect crosslinks between two or more collagen fibrils, including decorin and transglutaminase. In certain embodiments, a protein that crosslinks collagen fibers is not a hydroxylase, such as lysyl oxidase or prolyl oxidase. Although not a protein, riboflavin is also excluded from the practice of the invention.

"Transglutaminase" includes any of the individual transferase enzymes having the enzyme commission (EC) number EC 2.3.2.13. Examples of human transglutaminase proteins include those identified by the following REFSEQ numbers: NP_000350; NP_004604; NP_003236; NP_003232; NP_004236; NP_945345; and NP_443187. Besides human transglutaminase, transglutaminase prepared from non-human sources is included within the definition. Examples of non-human sources include, but are not limited to, primates, cows, pigs, sheep, guinea pigs, mice, and rats. Thus, in one illustrative example of subject-matter relating to the present invention, the transglutaminase is a transglutaminase solution prepared from an animal source (e.g., Sigma Catalogue No. T-5398, guinea pig liver). In other illustrative examples, however, the transglutaminase is from a recombinant source, and can be, for example, a human transglutaminase (e.g., the transglutaminase available from Axxora, 6181 Cornerstone Court East, Suite 103, San Diego, CA 92121 or from Research Diagnostics, Inc., a Division of Fitzgerald Industries Intl, 34 Junction Square Drive, Concord MA 01742-3049 USA.)

"Decorin" includes any of the proteins known to the skilled artisan by that name, so long as the decorin functions as a bidentate ligand attached to two neighboring collagen molecules in a fibril or in adjacent fibrils. In particular, decorin proteins include those proteins encoded by any of the various alternatively spliced transcripts of the human decorin gene described by REFSEQ number NM_001920.3. In general, the human decorin protein is 359 amino acids in size, and its amino acid sequence is set forth in REFSEQ number NP_001911. Various mutations and their effect on the interaction of decorin with collagen have been described, for example by Nareyeck et al., Eur. J. Biochem., Vo. 271, pages 3389-98 (2004), and those mutants that bind collagen are also within the scope of the term "decorin," as is the decorin variant known as the 179 allelic variant, De Cosmo et al., Nephron, Vol. 92, pages 72-76 (2002). Decorin for use in the methods of the invention may be from various animal sources, and it may be produce recombinantly or by purification from tissue. Thus, not only human decorin, but decorin from other species, including, but not limited to, primates, cows, pigs, sheep, guinea pigs, mice, and rats, may also be used in the methods of the invention. An example of human decorin that can be used in the methods of the invention is the recombinant human decorin that is available commercially from Gala Biotech. Glycosylated or unglycosylated forms of decorin can be used.

As used herein, the terms "treatment," "treating," and the like, refer to obtaining a desired pharmacologic and/or physiologic effect. A treatment can administer a composition or product to a patient already known to have a condition. A treatment can also administer a composition or product to a patient as part of a prophylactic strategy to inhibit the development of a disease or condition known to be associated with a primary treatment. In the context of a surgical procedure, prophylactic treatment is any treatment administered to a patient scheduled to undergo a surgical procedure for the purpose of improving the outcome of that surgical procedure or otherwise reducing undesirable secondary effects associated with the surgical procedure. An example of such a prophylactic treatment is the administration of an immunosuppressive agent to a patient prior to the transplantation of an organ or tissue. "Treatment," as used herein, covers any treatment of a condition or disease in a mammal, particularly in a human, and includes: (a) inhibiting the condition or disease, such as, arresting its development; and (b) relieving, alleviating or ameliorating the condition or disease, such as, for example, causing regression of the condition or disease.

A "pharmaceutically acceptable carrier" refers to a non-toxic solid, semisolid or liquid filler, diluent, encapsulating material or formulation auxiliary of any conventional type. A "pharmaceutically acceptable carrier" is non-toxic to recipients at the dosages and concentrations employed and is compatible with other ingredients of the formulation. For example, the carrier for a formulation containing polypeptides preferably does not include oxidizing agents and other compounds that are known to be deleterious to polypeptides. Suitable carriers include, but are not limited to, water, dextrose, glycerol, saline, ethanol, and combinations thereof. The carrier may contain additional agents such as wetting or emulsifying agents, pH buffering agents, or adjuvants which enhance the effectiveness of the formulation. Topical carriers include liquid petroleum, isopropyl palmitate, polyethylene glycol, ethanol (95%), polyoxyethylene monolaurate (5%) in water, or sodium lauryl sulfate (5%) in water. Other materials such as anti-oxidants, humectants, viscosity stabilizers, and similar agents may be added as necessary.

Pharmaceutically acceptable salts suitable for use herein include the acid addition salts (formed with the free amino groups of the polypeptide) and which are formed with inorganic acids such as, for example, hydrochloric or phosphoric acids, or such organic acids as acetic, mandelic, oxalic, and tartaric. Salts formed with the free carboxyl groups may also be derived from inorganic bases such as, for example, sodium, potassium, ammonium, calcium, or ferric hydroxides, and such organic bases as isopropylamine, trimethylamine, 2-ethylamino ethanol, and histidine.

The terms "individual," "subject," "host," and "patient," used interchangeably herein, refer to a mammal, including, but not limited to, murines, simians, humans, felines, canines, equines, bovines, porcines, ovines, caprines, mammalian farm animals, mammalian sport animals, and mammalian pets.

Before the present invention is further described, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

**II. Proteins That Crosslink Collagen Fibrils**

Some proteins can crosslink collagen fibrils directly by forming covalent bonds between two or more collagen fibrils without the protein itself becoming part of the covalent bond. Transglutaminase, which includes any of the individual transferase enzymes having the enzyme commission (EC) number EC 2.3.2.13, is an example of a protein of this type. Transglutaminase catalyzes the formation of a covalent bond between a free amine group (e.g., on a lysine) and the gamma-carboxamide group of glutamine. Thus not itself part of the bond, transglutaminase instead forms a direct covalent link between two collagen fibrils.

In some illustrative examples of the invention, transglutaminase is prepared in 0.01 M Tris buffer, pH 7.2. But any other pharmaceutically acceptable buffer may be used so long as it does not form a complex with calcium and prevent activation of transglutaminase. Buffer concentrations therefore generally range from between 5mM to 100mM or from between 10mM and 50mM. In some illustrative examples of the invention, the buffer concentration is 10mM. The buffer may also include CaCl₂ in concentrations that range from 5mM to 50mM, or from 20mM to 35mM. In certain embodiments of the invention, the buffer is a 25mM CaCl₂ solution

Irrespective of the buffer solution chosen, when transglutaminase is the protein chosen, its concentration generally ranges from 1 to 100 units, but in some illustrative examples the concentration may be from 5 units to 50 units. In other illustrative examples of the invention, the concentration ranges from 10 units to 25 units per 50mL of final enzyme solution. An illustrative example of one transglutaminase solution of the is 0.01 M Tris buffer, pH 7.2, containing 25mM CaCl₂ and from 10 units to 25 units of transglutaminase per 50mL. Other proteins that catalyze formation of direct bonds between collagen fibrils may be used at these concentrations and in these buffers as well.

Collagen fibrils can also be crosslinked by indirect bonds. In these embodiments of the invention, one or more proteins serve as an intermediary link between or among the collagen fibrils. Decorin is an example of a protein that crosslinks collagen fibrils by indirect bonds.

For use in the invention, decorin is generally dissolved or suspended in a physiologically compatible buffer solution. The concentration of decorin may range from about 10 to about 1000 µg/ml. In some embodiments, the concentration ranges from about 50 to about 750 µg/ml, while in other embodiments it may be from about 100 to about 500 µg/ml. Other proteins that indirectly link collagen fibers by forming a bridge between or among collagen fibrils may be used at the concentrations described for decorin.

The buffer used as a carrier for a protein that forms an indirect crosslink between collagen fibrils is not critical and may be any of a number of pharmaceutically acceptable buffers, such as a neutral pH phosphate buffer. Other suitable buffers include HEPES, TRIZMA® (Sigma-Aldrich, but any other supplier of TRIS buffer should also be acceptable). The buffer will generally have a concentration from about 0.005 to 0.5M at a pH ranging from 6.5 to 8.5, although in some embodiments the pH is from about 6.8 to about 7.6.

An example of a decorin solution for use in the methods of the invention is one that is sterile and non-pyrogenic, and in which decorin is present at a concentration of 500 µg/ml and is buffered with 10mM sodium phosphate plus 15mM NaCl having a pH of 7.2.

**III. Method of Administering Proteins That Crosslink Collagen Fibrils to the Eye**

Various methods can be used to apply a protein that crosslinks collagen fibrils to the corneal surface. Although the actual methods may vary, what is important is that the protein is applied to a controlled area of the corneal surface. Thus, in an illustrative example of subject matter relating to the present invention, a solution comprising a protein that crosslinks collagen fibrils is applied to an applicator that is positioned on the corneal surface. A reservoir in the applicator allows the protein solution to penetrate a controlled area of the corneal surface. The reservoir also prevents the protein solution from flowing off of the corneal surface and onto surrounding ocular tissues.

As an illustrative example, when transglutaminase is used, it may be prepared using the following procedure. An inactive enzyme preparation is first prepared. For example, 10 units of transglutaminase can be added to 10mL of Tris buffer, and mixed until the transglutaminase crystals dissolve. The resulting solution can then be diluted to 50mL by adding sterile water and stored frozen until ready to use. Activate enzyme may then be prepared by the addition of CaCl₂. Usually this is done just before application to corneal tissue. For example, 1 part CaCl₂ solution can be added to 10 parts transglutaminase solution. One mL of transglutaminase solution is usually sufficient for each application.

The methods have been described generally with respect to their method steps and the compositions used. Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

It must be noted that as used herein and in the appended claims, the singular forms "a", "an" and "the" include plural referents unless the context clearly dictates otherwise. Thus, for example, reference to "a subject polypeptide" includes a plurality of such polypeptides and reference to "the agent" includes reference to one or more agents and equivalents thereof known to those skilled in the art, and so forth.

Unless defined otherwise, all technical and scientific terms used herein have the same meaning as commonly understood by one of ordinary skill in the art to which this invention belongs. All publications mentioned herein, including patents, patent applications, are incorporated herein by reference in their entireties to disclose and describe the methods and/or materials in connection with which the publications are cited.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

The invention described below is given by way of example only and is not to be interpreted in any way as limiting the invention.

Reference will now be made in detail to the present embodiments of the invention.

Example 1: Transglutaminase Stabilizes the Shape of the Cornea Following Mechanical Deformation

Transglutaminase was studied in a series of *ex vivo* laboratory experiments on enucleated porcine cornea to optimize the effects of stabilization. Enucleated porcine eyes were placed in ice until treated. Prior to treatment, each eye was placed in a bracket for stability and subjected to topographical evaluation using the Optikon 2000 system. Six topographs of each eye were taken and true composites generated. The corneal surface was dried using sterile gauze and then wetted with drops of 0.02M disodium phosphate. The wetted eyes were again dried and exposed to drops of 0.02M disodium phosphate. A glass slide was balanced on the surface of the cornea. Solutions of transglutaminase and calcium chloride (CaCl₂) were prepared in 50mM TRIS buffer, pH 8.5. The pH of TRIS buffer was adjusted to 8.5 by adding 2.5N sodium hydroxide (NaOH). Transglutaminase was prepared at 1 mg/mL in 10mL of TRIS buffer. CaCl₂ was prepared at a concentration of 25mM in 50mL of TRIS buffer. Prior to administration, 1mL of CaCl₂ solution was mixed with 9mL of transglutaminase solution because transglutaminase requires Ca⁺⁺ as a catalyst. The transglutaminase/CaCl₂ solution was added dropwise to the area around the glass slide. Approximately 1 mL of enzyme solution was applied in a period of 2 minutes. The slide was then removed and the eye washed with 0.004M phosphate buffer, at pH 7.4. The eye was then reexamined topographically and photos taken. Following the topographical evaluation, the eyes were placed in Optisol for storage pending additional evaluations. Three eyes were treated using this protocol.

There were some difficulties in treating the first two eyes due to the difficulty in applying the enzyme solution while balancing the glass slide. In the third attempt, drops of transglutaminase were applied to the cornea and the slide applied to the corneal surface and held in place using thumb pressure. Drops of enzyme solution were subsequently applied to corneal surfaces around the glass slide. No flattening effect was noted in the first two eyes. The topographical results from the first eye appeared to show corneal steepening as shown in Table 1 below. However, topographical maps clearly demonstrated a flattening of the central cornea in the third eye. Refractive power was reduced by approximately 1.5 diopters. All eyes appeared clear by visual examination. Eyes were placed in Optisol for storage.

**Table 1: Corneal Power as Measured by Topographical Mapping**

| Porcine Eye No. | Pretreatment (in Diopter) | | Post-treatment (in Diopter) | |
|---|---|---|---|---|
| 1 | 38.98 | 37.33 | 42.14 | 39.2 |
| 2 | 39.89 | 37.7 | 39.98 | 37.26 |
| 3 | 40.25 | 38.16 | 38.79 | 36.83 |

After treatment, corneal buttons were dissected, placed in Optisol and shipped to Rutgers University for stress-strain analysis. In the stress-strain analysis, corneal buttons were placed on a slightly convex surface and exposed to compressive forces. Stress-strain curves represent the force per unit area of cross-section required to compress the cornea a certain amount as expressed in percentage. Resultant curves indicate several distinct phases. The lower part (low modulus region) represents the resistance to squeeze out fluid between collagen fibrils. The middle part, wherein the stress-strain curve does not change, and the upper part (high modulus region) represent compression of collagen fibrils. A reduction in low modulus indicates that the cornea is softer. An increase indicates that the corneal buttons are stiffer and have been stabilized.

Transglutaminase treatment gave encouraging results. Topographical evaluation indicated that one porcine eye treated with transglutaminase following corneal flattening using a glass slide exhibited a refractive power reduction of about 1.5 diopters after removal of the glass slide. Two additional eyes were included in this treatment series. Glass slides were also applied to these porcine eyes. However, enzyme addition was applied with the eyes in the horizontal position and did not appear to flow under the glass slide into the cornea. In these eyes, there was no evidence of a reduction in refractive power by topographical evaluation. Since these eyes did not show flattening of the central cornea following the application of the glass slide, it was unlikely that the corneal flattening observed in the successful eye was solely a result of the application of the glass slide.

Example 2. Toxicity Evaluation of Decorin in the Feline Eye

The purpose of the following evaluation was to determine if (1) there is toxicity associated with the use of decorin on the eye; (2) assess the penetration of decorin into the cornea; and (3) quantitate decorin in the cornea following exogenous application of decorin.

One, three, and five daily applications of decorin were assessed using female cats (6 months to 2 years of age) with normal corneas as the model system. The decorin was obtained as a dry powder (Sigma Chem. Co., Milwaukee, WI) and reconstituted in a 0.1 M phosphate buffer. In order to perform the microscopic evaluations, decorin was labeled with Oregon Green 514 using a commercially available kit from Molecular Probes.

Five cats were used in the study. Each cat was sedated prior to topical application of medication or photography of the eye. All animals received an ocular examination and photographs (whole eye, slit lamp, and endothelial cells) prior to treatment. Eyes were randomly assigned to a treatment group. The decorin was applied to the interior of a contact lens and the lens placed on the cat's eye. The lens remained on the eye for 10 minutes. All animals were observed briefly daily during the study. Three eyes were randomly assigned to a treatment or control group (1 eye). At least 2 more eyes were obtained for use as controls for each of the histograph, TEM, and confocal microscopy evaluations.

One eye from each of the three treatment groups was treated with Oregon Green 5149 labeled decorin.

Treatment group 1 eyes received one application of 50µg of decorin in 100µl buffer on day 1. Photographs and exams were obtained just after treatment and again on days 2, 4, and 8-post treatment. Exams and photos were then done weekly for the remainder of the month.

Treatment group 2 eyes received one application of 50µg decorin in 100µl buffer on days 1, 2, and 3. Photographs and exams were obtained just after treatment and again on days 2, 3, 5, and 8. Thereafter, exams and photos were obtained weekly for the remainder of the month.

Treatment group 3 eyes received one application of 50µg decorin in 100µl of buffer on days 1, 2, 3, 4, and 5. Photographs and exams were obtained daily and again on day 8. Thereafter, exams and photos were obtained weekly for the remainder of the month.

All animals were euthanized at one month. The eyes were enucleated. Each eye was cut in half. One half was fixed in formalin for histolgical analysis (H&E stain) of toxicity. The second half was further divided in half, one section was used for TEM visualization of the decorin, and the remainder was examined using confocal microscopy for those cats treated with labeled decorin.

The confocal micrograph results showed that decorin penetrates the corneal tissue of the eye. In addition, a cornea treated five times with decorin according to the treatment protocol above (treatment group 3) contained more collagen fibril-associated decorin than the untreated cornea. Initial qualitative analysis indicates that the decorin filaments in the treated eye appear longer and "fatter". These "fatter" filaments were observed throughout the stromal sections, including the epithelial region, mid-stroma, and endothelial regions.

In trials with live human subjects in Shanghai, People's Republic of China in August, 2004, 2.9 mg/ml of decorin in buffered saline solution was administered by the applicator method. To avoid discomfort from placing the applicator on the eye, Proparican Hydrochloride (0.5%) was first administered as an anesthetic.

While the present invention has been described with reference to the specific embodiments thereof, it should be understood by those skilled in the art that various changes may be made and equivalents may be substituted without departing from the scope of the invention. In addition, many modifications may be made to adapt a particular situation, material, composition of matter, process, process step or steps, to the objective, and scope of the present invention. All such modifications are intended to be within the scope of the claims appended hereto. Other embodiments of the invention will be apparent to those skilled in the art from consideration of the specification and practice of the invention disclosed herein. It is intended that the specification and examples be considered as exemplary only, with a true scope of the invention being indicated by the following claims.

## Claims

1. Decorin for use in improving an outcome following a refractive surgical procedure wherein:
a) a patient is to undergo a refractive surgical procedure and the decorin is administered to the eye of the patient before the procedure is carried out, and/or
b) the decorin is administered to the eye of the patient during a refractive surgical procedure, and/or
c) the decorin is administered to the eye of the patient following a refractive surgical procedure.

2. Decorin for use as claimed in claim 1 for use in treating or preventing post-operative keratectasia.

3. Decorin for use as claimed in Claim 1 or Claim 2, wherein the refractive surgical procedure is one of radial keratotomy (RK), photorefractive keratectomy (PRK) or laser in-situ keratomileusis (LASIK).

4. Decorin for use in preventing or treating keratectasia or keratoconus by administration to the eye of a patient who has keratectasia or keratoconus.

5. Decorin for use as claimed in Claim 4, wherein the keratectasia develops following a refractive surgical procedure.

6. Decorin for use as claimed in Claim 5, wherein the keratectasia develops following laser in-situ keratomileusis (LASIK).

7. Decorin for use as claimed in any preceding claim wherein the decorin is glycosylated or unglycosylated.

8. Decorin for use as claimed in any preceding claim which is a recombinant human decorin.

## Patentansprüche

1. Decorin für die Verwendung zur Verbesserung eines Ergebnisses nach einem refraktiv-chirurgischen Eingriff, worin:
a) ein Patient sich einem refraktiv-chirurgischen Eingriff unterzieht und das Decorin dem Auge des Patienten verabreicht wird, bevor der Eingriff durchgeführt wird, und/oder
b) das Decorin dem Auge des Patienten während eines refraktiv-chirurgischen Eingriffs verabreicht wird, und/oder
c) das Decorin dem Auge des Patienten nach einem refraktiv-chirurgischen Eingriff verabreicht wird.

2. Decorin für die Verwendung nach Anspruch 1 für die Verwendung zur Behandlung oder Prävention von post-operativer Keratektasie.

3. Decorin für die Verwendung nach Anspruch 1 oder Anspruch 2, worin der refraktiv-chirurgische Eingriff einer von radialer Keratotomie (RK), photorefraktiver Keratektomie (PRK) oder Laser-In-Situ-Keratomileusis (LASIK) ist.

4. Decorin für die Verwendung zur Prävention oder Behandlung von Keratektasie oder Keratokonus durch Verabreichung an das Auge eines Patienten, der Keratektasie oder Keratokonus hat.

5. Decorin für die Verwendung nach Anspruch 4, worin sich die Keratektasie nach einem refraktiv-chirurgischen Eingriff entwickelt.

6. Decorin für die Verwendung nach Anspruch 5, worin sich die Keratektasie nach Laser-In-Situ-Keratomileusis (LASIK) entwickelt.

7. Decorin für die Verwendung nach einem vorstehenden Anspruch, worin das Decorin glycosyliert oder nicht glycosyliert ist.

8. Decorin für die Verwendung nach einem vorstehenden Anspruch, das ein rekombinantes menschliches Decorin ist.

## Revendications

1. Décorine destinée à une utilisation pour améliorer le résultat clinique suite à une opération chirurgicale réfractive, dans laquelle :
a) un patient doit subir une opération chirurgicale réfractive et la décorine est administrée à l'oeil du patient avant la mise en oeuvre de l'opération, et/ou
b) la décorine est administrée à l'oeil du patient pendant la mise en oeuvre d'une opération chirurgicale réfractive, et/ou
c) la décorine est administrée à l'oeil du patient après la mise en oeuvre d'une opération chirurgicale réfractive.

2. Décorine destinée à une utilisation selon la revendication 1, pour une utilisation dans le traitement ou la prévention d'une kératectasie post-opératoire.

3. Décorine destinée à une utilisation selon la revendication 1 ou la revendication 2, dans laquelle l'opération chirurgicale réfractive est choisie parmi la kératotomie radiaire (RK), la kératectomie photoréfractive (PRK) ou un kératomileusis laser *in situ* (LASIK).

4. Décorine destinée à une utilisation pour empêcher ou traiter une kératectasie ou un kératocône, par administration à l'oeil d'un patient souffrant d'une kératectasie ou d'un kératocône.

5. Décorine destinée à une utilisation selon la revendication 4, dans laquelle la kératectasie se développe suite à une opération chirurgicale réfractive.

6. Décorine destinée à une utilisation selon la revendication 5, dans laquelle la kératectasie se développe suite à un kératomileusis laser *in situ* (LASIK).

7. Décorine destinée à une utilisation selon l'une quelconque des revendications précédentes, dans laquelle la décorine est glycosylée ou non glycosylée.

8. Décorine destinée à une utilisation selon l'une quelconque des revendications précédentes, qui est une décorine humaine recombinée.
